# DEMANDE DE BREVET EUROPEEN

(11) **EP 1 346 725 A1**
(43) Date de publication de la demande: **24.09.2003**
(21) Numéro de dépôt: 03013631.1
(22) Date de dépôt: 19.04.2000
(51) Int. Cl.: A61K 35/16, A61K 31/7032, A61P 35/00, A61P 31/04, C12N 5/06

(54) **Composition pharmaceutique comprenent des cellules NKT activées par des PIM, et son utilisation en thérapie**

(30) Priorité: 19.04.1999 FR 9904897
(62) Demande divisionnaire de: 00920847.1
(71) Demandeur: INSTITUT NATIONAL DE LA SANTE ET DE LA RECHERCHE MEDICALE (INSERM), 75654 Paris Cédex 13 (FR); INSTITUT PASTEUR, 75724 Paris Cédex 15 (FR)
(72) Inventeur: Apostolov, Irina, 75020 Paris (FR); Gachelin, Gabriel, 75012 Paris (FR); Kourilsky, Philippe, 75001 Paris (FR); Takahama, Yousuke, Kokofu, Tokushima 779-3118 (JP)
(74) Mandataire: Bernasconi, Jean Raymond

(57) **Abrégé**

Cette invention concerne une composition pharmaceutique comprenant au moins une cellule NKT activée par un PIM, et l'utilisation d'au moins un PIM et/ou au moins une cellule NKT activée par un PIM pour le traitement d'une maladie pour lequel une réaction immunitaire du type granulomateuse est désirée.

## Description

La présente invention concerne l'utilisation d'au moins un PIM et/ou au moins une cellule NKT activée par un PIM pour le traitement d'une maladie pour lequel une réaction immunitaire du type granulomateuse est désirée.

Les cellules NKT sont un sous-groupe particulier de lymphocytes T α/β exprimant des marqueurs de surface cellulaire associés aux cellules NK ("Natural Killer"), telles que NK1.1 (Bendelac et al, 1995) et certains membres de la famille Ly-49 (Ortaldo et al, 1998). Elles diffèrent également des lymphocytes T conventionnels par plusieurs caractéristiques de leurs récepteurs T : la chaîne α est invariante et résulte d'un réarrangement entre les segments Vα14 et Jα281 associés à une région conservée de CDR3 (Koseki et al, 1991 ; Lantz et al, 1994). Ils utilisent en outre un répertoire TCRβ restreint (Bendelac et al, 1997). Enfin, et contrairement aux cellules T conventionnelles, les cellules NKT expriment le récepteur TCR (récepteur des cellules T) à un niveau intermédiaire et sont restreints par la molécule du CMH de classe 1b, CD1 (Bendelac et al, 1997). Les domaines α1 et α2 de la molécule CD1d1 présentent un site de liaison hydrophobe qui s'accommode du noyau lipidique des glycosylcéramides (Brossay et al, 1998) et des protéines ancrées par un GPI (glycosylphosphatidylinositol), créant des complexes CD1-glycolipides qui sont reconnus par les cellules NKT (Brossay et al, 1998 ; Kawano et al, 1997 ; Burdin et al, 1998 ; Brossay et al, 1998, Schofield et al, 1999).

Les fonctions *in vivo* des cellules NKT sont loin d'être complètement décrites. Les cellules NKT jouent un rôle dans le rejet des tumeurs médiées par l'IL-12 (Takeda et al, 1996 ; Cui et al, 1997 ; Kawano et al, 1998). Elles sont également impliquées dans certaines maladies autoimmunes humaines et murines et des processus infectieux, soit par la production de cytokine de type Th-1 ou Th-2 (Hammond et al, 1998 ; Flesch et al, 1997 ; Enomoto et al, 1997) ou par une baisse du nombre de ces cellules (Mieza et al, 1996 ; Wilson et al, 1998; Emoto et al, *Eur. J. Immunol*. 1997 ; Emoto et al, *Inf. & Imm*., 1997).

Les mécanismes qui initient l'activation *in vivo* des cellules NKT sont également très mal connus. Une activation de ces cellules par un céramide, l'α-galactosylcéramide a été mise en évidence (Kawano et al, 1998 et WO 98/44 928). Un article récent de Schofield et al, 1999 rapporte par ailleurs les résultats de la réponse des cellules NKT périphériques à divers GPI (glycophosphatidylinositol) *in vivo.*

Les auteurs de la présente invention ont découvert d'une part, que les cellules NKT peuvent être activées par des phosphoinositolmannosides (PIM) et d'autre part, que cette activation provoque une réaction immunitaire de type granulomateuse.

Les auteurs de la présente invention ont également mis en évidence que l'activation des cellules NKT responsables de la réponse granulomateuse est indépendante de la voie CD1/TCR et dépend en fait de la voie d'immunité innée médiée par CD14. Les PIMs activant les cellules NKT permettraient d'orienter la réponse de l'hôte vers une voie de type TH1. Ceci a pu être montré par les inventeurs à l'aide de mesures directes des cytokines relarguées au sein du granulome.

Sur la base de ces résultats, les auteurs de la présente invention proposent d'utiliser au moins un PIM et/ou au moins une cellule NKT activée par un PIM pour le traitement d'une maladie pour lequel une réaction immunitaire du type granulomateuse est désirée.

Conformément à la présente invention, on peut utiliser le phosphotidylinositol mannoside (PIM), en association avec un véhicule pharmaceutiquement acceptable.

Le phosphatidylinositolmannoside utilisé peut être obtenu par synthèse chimique, par exemple à partir du phosphate d'inositol, par réaction avec un diacylglycérol puis mannosylation, selon les méthodes connues. Les PIM utilisés peuvent également être obtenus par purification à partir des parois de bactéries.

La purification des PIM à partir des parois de bactéries peut être réalisée suivant les techniques standards connues de l'homme du métier. De préférence, le PIM utilisé est d'origine mycobactérienne et peut être par exemple obtenu à partir de *Mycobacterium tuberculosis*.

Parmi les PIM que l'on peut utiliser, on peut citer ceux qui répondent à la formule : 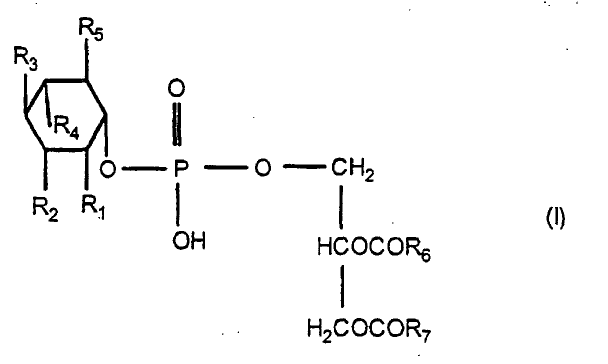 dans laquelle :
- R₁, R₂, R₃, R₄ et R₅ sont, indépendamment l'un de l'autre, choisis parmi un groupement hydroxyle et un groupement (Man)ₓ,
où (Man)ₓ représente au moins un groupement mannoside lié à l'inositol, x étant le nombre de motifs de mannose (reliés entre eux de manière linéaire), qui varie de préférence de 1 à 6 ;
- R₆ ou R₇ sont, indépendamment l'un de l'autre, un reste d'acide gras, de préférence comportant de 18 à 24 atomes de carbone, et de préférence ne comportant pas de double liaison, et de préférence encore, ne comportant pas de substituant polaire.

De manière préférentielle, R₁, R₂, R₃, R₄ et R₅ sont tels que seuls deux d'entre eux, de préférence adjacents, représentent un groupement (Man)ₓ, les trois autres groupements étant alors un groupement hydroxyle.

De préférence, les PIM utilisés sont les PIMs de formule (I) dans laquelle R₂ et R₃ sont des groupements (Man)ₓ et R₁, R₄ et R₅ sont des groupements OH.

Peuvent par exemple être utilisés des PIM de formule (I), dans laquelle :
R₁, R₄ et R₅ sont des groupements OH ;
R₂ est un groupement mannoside [(Man)ₓ avec x=1] ; et
R₃ est (Man)ₓ avec x de 1 à 6.

Il est également possible d'utiliser un PIM modifié de telle sorte qu'il ne contienne non pas deux mais un seul reste d'acide gras.

Conformément à la présente invention, les PIM comportant 4, 5 ou 6 motifs mannose sont préférés.

La présente invention fournit également une composition pharmaceutique comprenant au moins une cellule NKT activée par un PIM, en association avec un véhicule pharmaceutiquement acceptable.

Les auteurs de la présente invention ont découvert que les cellules NKT activées par un PIM se distinguent des cellules NKT activées par exemple par un céramide. L'activation des cellules NKT par un PIM serait en fait relativement spécifique et impliquerait une distribution oligoclonale. En revanche, l'activation des cellules NKT par exemple par un céramide serait polyclonale, et ne provoquerait pas de réponse granulomateuse intense.

Conformément à la présente invention, l'activation des cellules NKT par un PIM peut être réalisée *in vivo* ou *ex vivo.*

La présente invention fournit donc également une méthode d'activation des cellules NKT *in vivo* comprenant les étapes consistant à :
i) administrer au moins un PIM, en association avec un véhicule pharmaceutiquement acceptable, à un sujet ;
ii) prélever chez ledit sujet un échantillon biologique contenant des cellules NKT, lesdites cellules ayant été activées *in vivo* par ledit PIM.

Les cellules NKT activées par un PIM *in vivo* peuvent ensuite être administrées à un sujet nécessitant un tel traitement, qui peut être le sujet auquel l'échantillon biologique a été prélevé ou un autre sujet. Une telle méthode permet notamment d'injecter ces cellules NKT activées à un site particulier d'un sujet où une réponse granulomateuse est désirée.

La présente invention fournit également un procédé d'activation de cellules NKT *ex vivo* comprenant les étapes consistant à :
i) prélever un échantillon biologique contenant des cellules NKT ;
ii) mettre en contact les cellules NKT avec au moins un phosphatidylinositolmannoside (PIM), dans des conditions appropriées pour que le PIM active les cellules NKT.

Les cellules NKT activées par un PIM *ex vivo* peuvent être alors administrées à un sujet nécessitant un tel traitement. Une telle méthode permet notamment d'injecter ces cellules NKT activées à un site particulier d'un sujet où une réponse granulomateuse est désirée.

La présente invention fournit donc également une méthode de traitement thérapeutique d'une affection pour lequel une réaction immunitaire de type granulomateuse est désirée, ladite méthode comprenant l'administration à un sujet nécessitant un tel traitement d'une quantité thérapeutiquement efficace d'au moins un PIM et/ou de cellules NKT activées par un PIM.

La présente invention concerne également l'utilisation d'au moins un phosphatidylinositolmannoside (PIM) et/ou au moins une cellule NKT activée par un PIM pour la fabrication d'un médicament destiné au traitement d'une maladie pour lequel une réaction immunitaire de type granulomateuse est désirée.

Par "traitement", on entend toute intervention à visée thérapeutique curative mais non préventive.

Ladite maladie peut être notamment une maladie ayant pour origine une infection par des agents bactériens, par exemple des mycobactéries, telles que les mycobactéries responsables de la lèpre et de la tuberculose. Les PIM ou les cellules NKT activées par les PIM agissent en stimulant l'immunité locale, et en particulier l'immunité mucosale lors d'une infection bactérienne.

Les modes d'administration, les posologies et les formes galéniques des compositions pharmaceutiques selon l'invention peuvent être déterminés de manière usuelle par l'homme du métier, notamment selon les critères généralement pris en compte pour l'établissement d'un traitement thérapeutique adapté à un patient, comme par exemple l'âge ou le poids corporel du patient, la gravité de son état général, la tolérance au traitement, etc.

De manière avantageuse, une composition pharmaceutique selon l'invention, contenant un PIM ou des cellules NKT activées par un PIM, peut être administrée par voie intraveineuse ou sous-cutanée par exemple.

Le médicament selon l'invention peut notamment être utile pour le traitement de cancers, comme par exemple les mélanomes qui sont connus pour régresser lors de réponses granulomateuses intenses. Dans ce cas, les PIM ou les cellules NKT activées par des PIM peuvent être de manière avantageuse utilisées comme adjuvants à des agents anti-tumoraux connus.

Les exemples et figures suivants illustrent l'invention sans en limiter la portée de l'invention.

### LEGENDE DES FIGURES

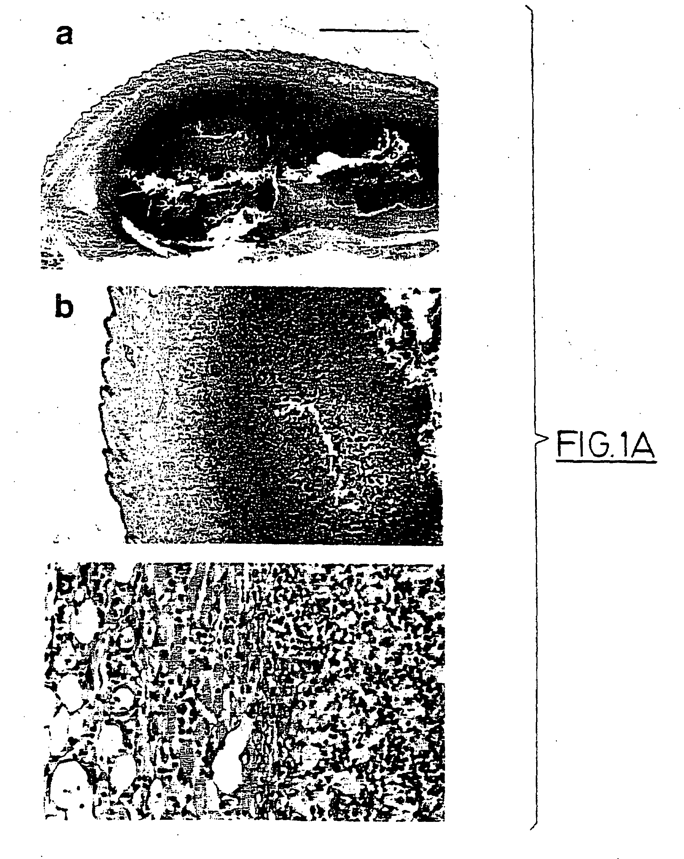

La figure 1A représente des sections colorées à l'hématoxylineéosine d'une structure de type granulomateuse causées par l'injection de parois cellulaires déprotéinisées de mycobactéries. On a injecté à des souris C57BL/6, par voie sous-cutanée l'équivalent de 10⁶ parois de cellules de mycobactéries déprotéinisées. Au jour 7 suivant l'infection, les structures de type granulomateuse qui se sont développées ont été excisées, fixées dans de la formaline tamponnée à 4 %, recouvertes de paraffine et sectionnées. Les objectifs utilisés sont 2,5 (a), 6,3 (b) et 40 (c) respectivement. La barre correspond à 1 mm.

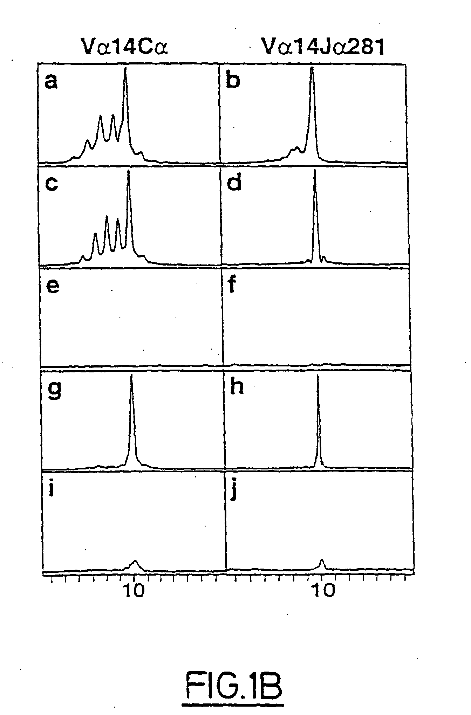

La figure 1B représente l'analyse par Immunoscope des profils Vα14-Cα (colonne de gauche) et Vα14-Jα281 (colonne de droite) observés sept jours après l'injection de PBS ou de parois de cellules de mycobactéries déprotéinisées à des souris C57BL/6 :
**a et b** : noyaux lymphatiques de souris auquels on a injecté du PBS.
**c** et **d** : noyaux lymphatiques de souris immunisées avec des parois cellulaires de mycobactéries déprotéinisées.
**e** et **f** : peau et muscle disséqués au site d'injection de PBS chez les souris contrôle.
**g** et **h** : structure de type granulomateuse au jour 7 causée par l'injection de parois de cellules de mycobactéries déprotéinisées.
**i** et **j** : infiltration cellulaire au jour 3.
abscisse : longueur en acides aminés de la région CDR3.
ordonnée : intensité relative de fluorescence.

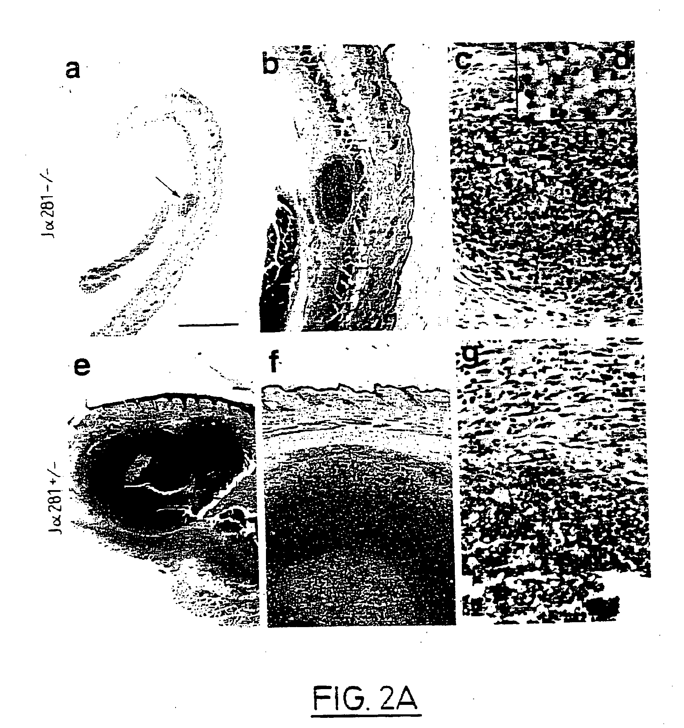

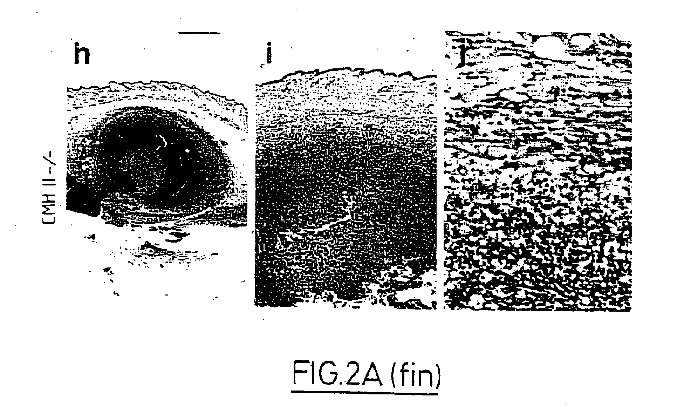

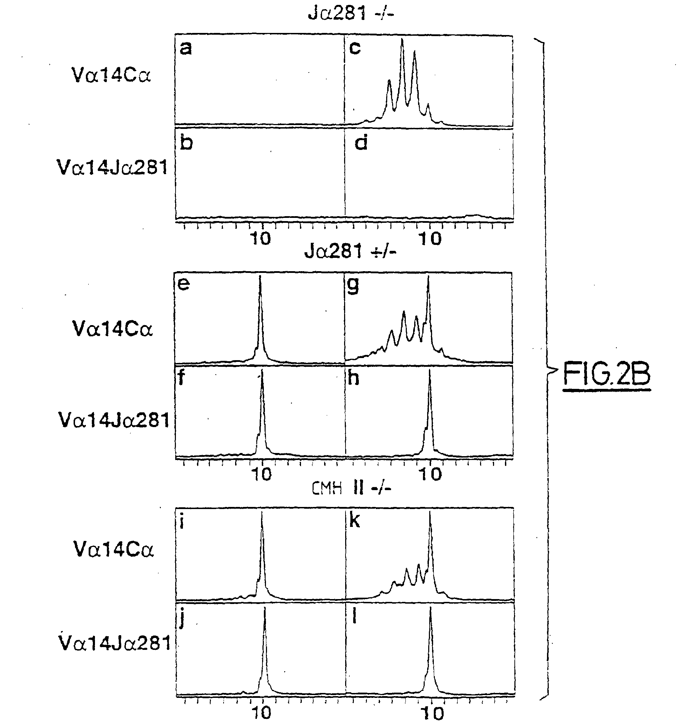

La figure 2 montre que le développement d'une structure du type granulomateuse de taille relativement importante dépend du recrutement de cellules NKT.

La figure 2A représente des sections colorées du site d'injection, 7 jours après l'injection des parois des cellules déprotéinisées chez les souris Jα281 -/- (objectifs : **a** 2,5, **b** 6,3, **c** 40 et **d,** huile, 100), Jα281+/- (**e** 2,5, f 6,3 et **g** 40), et C57BL/6 CMH de classe II -/- (**h** 2,5, **i** 6,3 et **j** 40). La barre correspond à 1 mm.

La figure 2B représente l'usage Vα14 et Vα14-Jα281 au site d'injection (colonnes de gauche) et dans les noyaux lymphatiques (colonnes de droite) de Jα281 -/- (**a** à **d**), Jα281 +/- (**e** à **h**) et C57BL/6 CMH de classe II -/- (**i** à **I**). Les ordonnées sont identiques à celles de la figure 1.

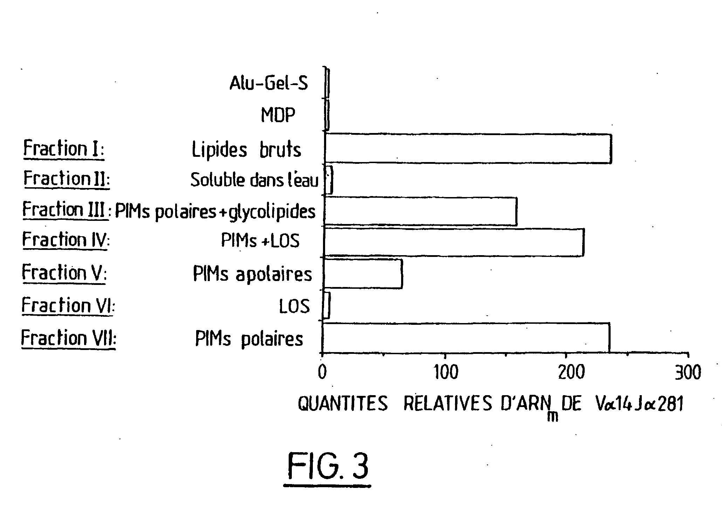

La figure 3 représente des quantités relatives d'ARNm Vα14 et Jα281 suivant l'immunisation avec Alu-Gel-S (hydroxyde d'aluminium) (0,25 mg), MDP (40 µg), les lipides mycobactériens bruts et des fractions obtenues à partir de ceux-ci. Le matériel contenu dans environ 10⁶ bactéries a été injecté après adsorption sur Alu-Gel-S. Les résultats (exprimés comme le rapport de la PCR fluorescente en utilisant des amorces Vα14-Jα281/ le signal de PCR fluorescent en utilisant des amorces hypoxanthine ribosyl transferase (HPRT) correspondent à la moyenne des résultats obtenus chez deux animaux.

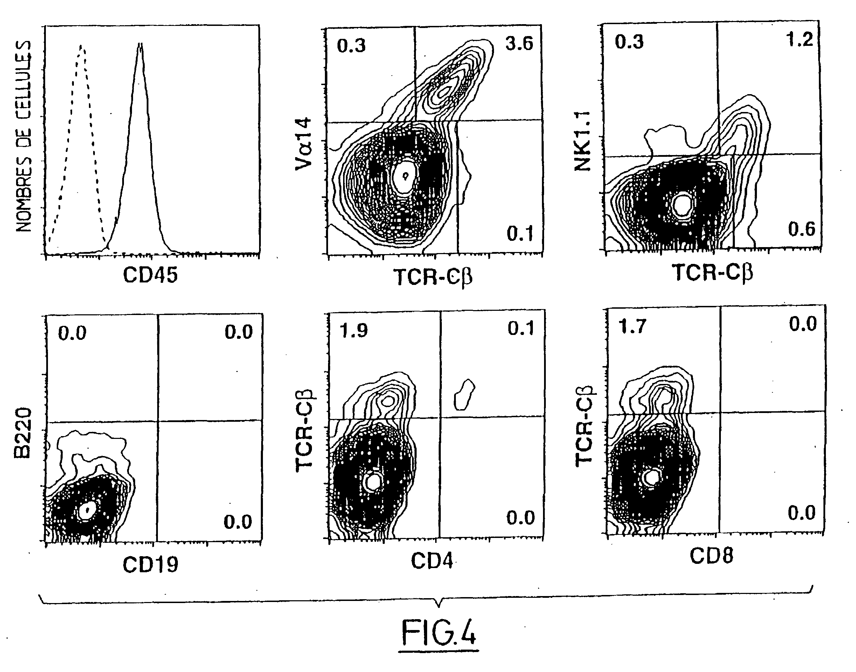

La figure 4 représente l'analyse par FACS des cellules recueillies au site de l'injection sous-cutanée de Alu-Gel-S/ lipides mycobactériens dans des souris C57BL/6. Selon les expériences, la fréquence des cellules NKT varie de 1,7 à 3,6 % des cellules totales recueillies après fractionnement par Ficoll. La ligne en pointillés sur l'histogramme correspond aux cellules non colorées.

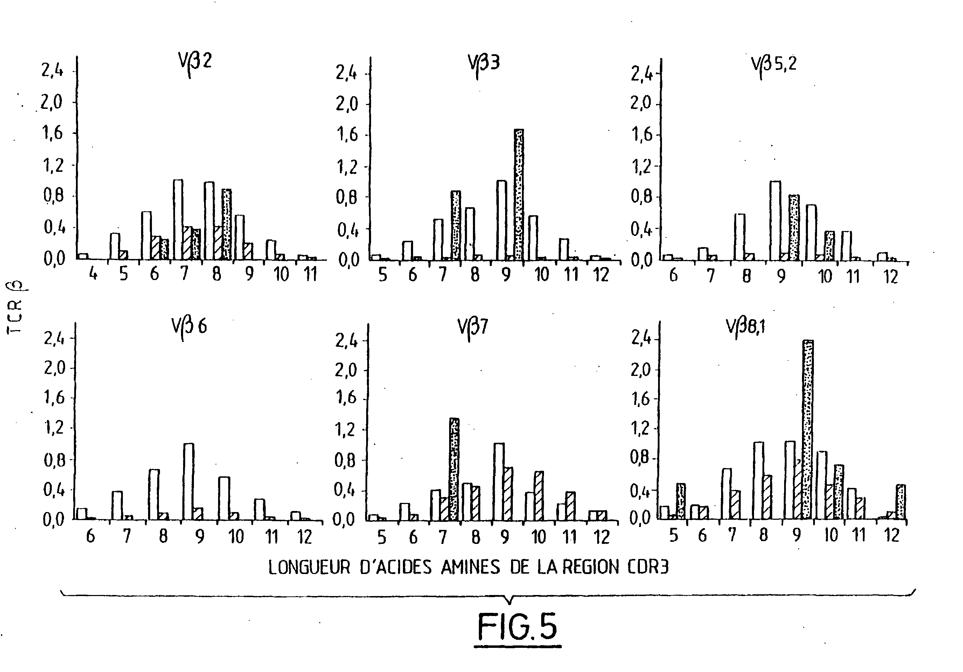

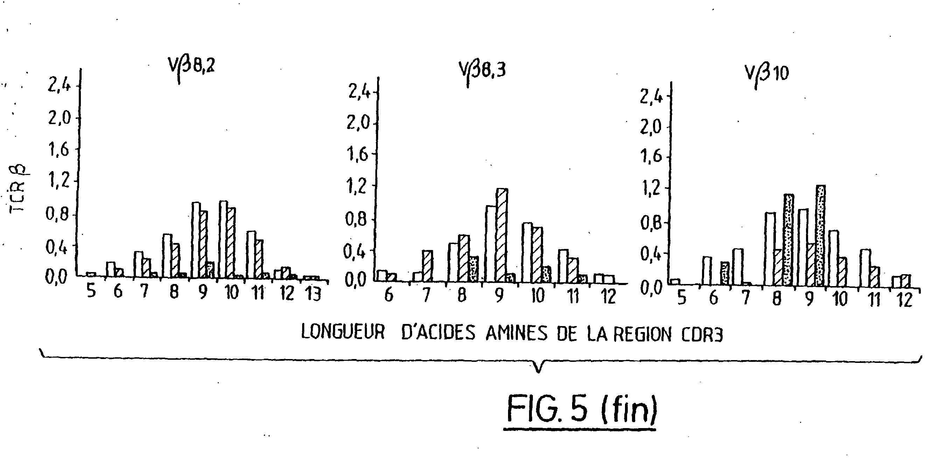

La figure 5 représente l'analyse semi-quantitative du répertoire de la chaîne TCRβ des cellules NKT isolées à partir du foie de souris contrôle (colonnes hachurées), recrutées par des PIM₄₋₆ (colonnes avec nuages de points) et de cellules T de noyaux lymphatiques utilisés ici comme contrôle interne pour l'efficacité des amorces (colonnes claires) : la valeur 1 est attribuée arbitrairement à l'aire du plus haut pic du profil gaussien obtenu à partir des cellules T du noyau lymphatique. Seules les familles les plus représentées de Vβ-Cβ dans les structures de type granulomateuse ont été étudiées par analyse PCR semi-quantitative. Vβ6, absent des structures de type granulomateuse, a été utilisé comme contrôle négatif.

### EXEMPLES

### MATERIELS ET METHODES

### Animaux.

Les animaux utilisés sont : souris C57BL/6 ; souris C57BL/6 de CMH de classe II -/- (Cosgrove et al, 1991) ; C57BL/6 β2m -/- (Koller et al, 1990) ; des souris Jα281-/- (Cui et al, 1997) et Jα281+/- ont été rétrocroisées respectivement 9 et 6 fois à partir de 129/Sv- dans le fonds génétique C57BL/6 au moment des expériences.

### Souches bactériennes et extraits :

On a cultivé sur un milieu semi-solide des bactéries *Mycobacterium tuberculosis* (souche H37Rv) que l'on a ensuite tuées par la chaleur (75°C, 30 minutes). Après sonication dans du Tris 10⁻²M, pH 8,0, MgCl₂ 10⁻³M, CaCl₂ 10⁻³M, NaCl 1,5x10⁻¹M, les mycobactéries ont été digérées pendant 5 heures à 37°C avec 25 mg/ml de DNAse et de RNAse. La digestion a été suivie d'une inactivation par la chaleur. Après plusieurs lavages, la préparation a été incubée pendant 24 heures, à 37°C avec un mélange de trypsine, chymotrypsine et subtilisine (Boehringer) à une concentration finale de 200 µg/ml pour chaque enzyme dans le même tampon que décrit précédemment et complétée par 0,5 % de SDS. Les enzymes ont été inactivées par la chaleur (10 minutes à 70°C). Les particules résultantes ont été lavées et resuspendues dans du tampon de phosphate (PBS). On a ensuite déterminé l'absence de protéines dans la suspension par électrophorèse sur PAGE et analyse d'acides aminés. Des échantillons ont été standardisés sur la base de leur contenu en sucre.

Les lipides mycobactériens ont été extraits et fractionnés comme suit : 4 g de bacilles H37Rv *M. tuberculosis* tués par la chaleur ont été extraits deux fois avec CHCl₃/CH₃OH (1:1, v/v). Les extraits ont été séchés, resuspendus dans CHCl₃/H₂O et laissés pendant une heure à 4°C afin que se distinguent une phase organique de lipides bruts (fraction I) et une phase aqueuse (incluant l'interface : LAM, polysaccharides et protéines dénaturées) (fraction II). La fraction I est dénuée de phosphoantigènes pour les cellules T γ/δ chez l'homme (Constant et al, 1994), mais comprend plusieurs composants glycolipidiques. Cette fraction a été séchée et séparée de nouveau par précipitation dans de l'acétone froid (10°C) pour donner un extrait soluble (graisse, glycolipides phénoliques) et un précipité blanc insoluble. De l'acétone chaud (50-60°C) a été ajouté au culot cireux, ce qui a permis de resuspendre les antigènes d'acide mycolique substitué par un tréhalose, et d'autres glycolipides (fraction III) et de précipiter la totalité des PIM et des lipooligosaccharides (LOS) (fraction IV). La fraction IV a été séchée et séparée par solubilisation au méthanol pour donner des PIM (culot blanc, fraction V) et des LOS solubles (fraction VI). Par refroidissement de la fraction III, quelques PIM polaires précipitent et donnent la fraction VII. Toutes les fractions ont été analysées par chromatographie sur couche mince (TLC) sur acide silicique pF254 (Merck) élué avec CHCl₃/CH₃OH/H₂O 60/35/5 et en utilisant un PIM2 de *M. tuberculosis* comme standard, les taches étant révélées en utilisant un spray anthrone-sulfurique.

### Amorces PCR :

Les amorces Vβ spécifiques, à l'exception de Vβ8.2 (Regnault et al, 1996) ont été décrites dans Pannetier et al, 1993. L'amorce Cβ spécifique non marquée utilisée pour la PCR est GCCCATGGAACTGCACTTGGC. L'amorce Cβ spécifique marquée est FAM-CTTGGGTGGAGTCACATTTCTC. Concernant l'ARNm des chaînes Vα14, les amorces suivantes ont été utilisées: l'amorce Vα14 était CTAAGCACAGCACGCTGCACA; l'amorce non marquée Cα était TGGCGTTGGTCTCTTTGAAG. L'amorce Cα spécifique marquée était FAM-ACACAGCAGGTTCTGGGTTC. L'amorce Jα281 spécifique non marquée était CAGGTATGACAATCAGCTGAGTCC. L'amorce Jα281 spécifique marquée était FAM-CAGCTGAGTCCCAGCTCC. L'amorce marquée spécifique clonotypique était FAM-GCTGAACCTCTATCNCCCACC. Les amorces pour CD4 étaient l'amorce 5' CTGAATTCGGCGCTTGCTGCTGC, l'amorce 3' CACAAGCTTAAGTCTGAGAGTCTTCC et FAM-TGCTGATTCCCCTTCCTTCC. Les amorces CD8 spécifiques étaient : l'amorce 5' TAGAATCCTAGCTTGACCTAAG, l'amorce 3' ATGGATCCATATAGACAACGAAGG et FAM-GGATAATCGACTCACCC. Les amorces pour HPRT étaient FAM-TTCTTTCCAGTTAAAGTTG, l'amorce 5' GTAATGATCAGTCAACGGGGGAC et l'amorce 3' CCAGCAAGCTTGCAACCTTAACCA. Les amorces pour CD3ε étaient : l'amorce 5' GCCTCAGAAGCATGATAAGC et 3'-FAM-CCCAGAGTGATACAGATGTC. Les amorces pour les chaînes lourdes des IgM étaient : FAM-TTCAGTGTTGTTCTGGTAG, l'amorce 3' CTGGATCCGGCACATGCAGATCTC, l'amorce 5' AGTCCTTCCCAAATGTCTTCCC. Les amorces non marquées Vγ1, Cγ, Vδ2, Vδ4, Vδ5, Vδ6 et Cδ spécifiques ont été décrites dans Azuara et al, 1997. Les amorces Vδ1- et les Vδ6P- spécifiques étaient respectivement ATTCAGAAGGCAACAATGAAAG et CTGTAGTCTTCCAGAAATCAC. L'amorce marquée Cδ spécifique était FAM-TTTCACCAGACAAGCAACA. Les amorces Vγ2- et Vγ7- spécifiques étaient respectivement CGGCAAAAAACAAATCAACA et CTATAACTTCGTCAGTTCCAC. L'amorce Vγ marquée spécifique des segments Vγ1 et Vγ2 était FAM-CCTCCTAAGGGTCGTTGATT et l'amorce marquée Vγ7- spécifique était FAM-CTTGTCCGGGCCTTCAT.

### Analyse semi-automatisée de la diversité des lymphocytes T ("Immunoscope") :

La technique basée sur la PCR utilisée par les auteurs de la présente invention est décrite dans Pannetier et al, 1997 ; Cibotti et al, 1994 ; Levraud et al, 1996. Brièvement, l'ARN total est extrait à partir des échantillons et 10 µg d'ARN total subit une transcription inverse en utilisant la transcriptase inverse AMV (virus de la myéloblastose aviaire). L'ADNc résultant est resuspendu dans un volume final de 100 µl.

La qualité et la quantité de l'ADNc ont été contrôlées par dosage de l'ARNm HPRT. 1 µl a été amplifié par 40 cycles de PCR (94°C, 1 min. ; 60°C, 1 min. ; 72°C, 4 min) en utilisant soit des amorces Vα14 et Cα spécifiques ou chacune des amorces Vβ et Cβ spécifiques. 2 µl des produits PCR ont été utilisés dans des extensions à partir d'amorces (5 cycles) en utilisant des amorces fluorescentes spécifiques des segments Jα281 ou Cα. La longueur de la région CDR3 et l'intensité de fluorescence de chaque famille des produits d'extension ont été déterminées par un sequenceur automatique (Applied Biosystems). La distribution de taille de la région CDR3 de chaque couple V-C est décrite comme une famille de pics séparés par 3 nucléotides (dérivés de l'ARNm en phase), dont l'aire est proportionnelle à l'intensité de la fluorescence et donc à la quantité initiale d'ADNc. En l'absence d'une stimulation antigénique spécifique, 6 à 8 pics avec une distribution de type gaussien ont été observés. En revanche, une réponse des lymphocytes T induite par un antigène résulte typiquement dans une distribution non gaussienne des pics due à une prolifération sélective des cellules portant certaines combinaisons particulières V-C. Une PCR semi-quantitative a été réalisée en utilisant une procédure adaptée à partir de celle décrite dans Azuara et al, 1997, et par normalisation sur la base de l'ARNm CD3ε. 10 000 lymphocytes T murins ont été suffisants pour réaliser une analyse par PCR semi-quantitative du répertoire Vβ-Cβ complet en utilisant la technique d'Immunoscope.

### Anticorps et analyse par FACS :

Cellules : Des cellules de granulomes ont été préparées en faisant passer des granulomes hachés à travers un filtre cellulaire en nylon (70 µm) ("strainer" Becton-Dickinson) et ont été lavées une fois dans du tampon phosphate (PBS). Les cellules vivantes ont été séparées par fractionnement Ficoll (Lymphocyte-M, Cedar Lane, Hornby, Ontario). Les leucocytes de foie ont été obtenus par gradient discontinu de Percoll.

Anticorps : le clone CMS-5 d'anticorps anti-Vα14 biotinylé a été décrit précédemment (Masuda et al, 1997). Les anticorps suivants ont été achetés auprès de Pharmingen (San Diego, CA) : PE-NK.1 (clone PK136), ou TCRβ biotinylé (clone H 57-597), FITC-CD4 (clone RM4-5), FITC-CD44 (clone IM7), CD45.2 biotinylé (clone 104), PE-B220 (clone RA3-6B2), FITC-CD19 (clone 1D3), FITC-CD8 (clone CT-CD8a). L'anticorps FITC-CD8 (clone CT-CD8a) a été obtenu auprès de CALTAG (San Francisco, CA). La PE-streptavidine a été obtenue auprès de Pharmingen, et l'APC-streptavidine a été obtenue auprès de Molecular probes. Les Ig de souris normales biotinylées FITC-, et PE- (phycoérythrine), utilisées comme contrôles négatifs, ont été achetées chez Pharmingen.

Analyse par FACS : Avant coloration avec des anticorps spécifiques, les cellules ont été incubées avec du sérum murin pour bloquer la coloration non spécifique. Diverses combinaisons d'anticorps ont ensuite été ajoutées. Les cellules mortes ont été mises à l'écart par coloration à l'iodure de propidium. Les cellules lymphoïdes ont été sélectionnées sur FCS et SSC. Un minimum de 100 000 événements a été compté dans la fenêtre des lymphocytes. Une analyse à quatre couleurs a été effectuée en utilisant un FACS-Vantage (Becton-Dickinson), et une analyse à trois couleurs a été réalisée en utilisant un FACS-Scan (Becton-Dickinson). Les données ont été réalisées en utilisant le logiciel Cell-Quest (Becton-Dickinson). Les cellules de foie NKT ont été triées après marquage avec des anticorps anti-NK1.1 et anti-TcRβ, en utilisant un trieur de cellules FACStar⁺ Becton-Dickinson.

### RESULTATS

### 1. L'injection de parois cellulaires déprotéinisées de mycobactéries résulte dans la formation de lésions de type granulomateuse.

Les auteurs de la présente invention ont tout d'abord examiné si les cellules NKT murines pouvaient être détectées au site d'injection de parois cellulaires déprotéinisées de mycobactéries, qui sont riches en glycolipides.

A cette fin, la suspension de parois cellulaires déprotéinisées de mycobactéries dans du PBS, équivalent à environ 10⁶ bactéries virulentes *Mycobacterium tuberculosis,* souche H37rv, a été injectée par voie sous-cutanée dans la base de la queue de souris C57BL/6. Les animaux à qui on a injecté du PBS ont été sacrifiés aux jours 1, 2, 3 et 7 suivant l'immunisation et aucun signe de processus inflammatoire n'a pu être observé au site d'injection du PBS. Chez les souris immunisées, des neutrophiles ont été observés de manière prédominante sur des frottis préparés à partir des infiltrats cellulaires recueillis aux jours 1 et 2. Les lymphocytes et macrophages ont été détectés au jour 3 et des lésions adhérant à la peau et aux muscles se sont développées par la suite. Ils ont augmenté en taille avec le temps, atteignant un diamètre de 4-6 mm au jour 7. Une analyse histologique a été effectuée sur les lésions induites au jour 7. Des sections colorées ont révélé des lésions de type granulomateuse, avec un coeur riche en neutrophiles entouré par une bordure consistant en une rangée dense de macrophages, lymphocytes et fibroblastes (figure 1A).

### 2. Les cellules NKT sont les seules cellules T Vα14⁺ qui infiltrent les lésions granulomateuses.

Les structures de type granulomateuse au jour 7 ne pouvaient être dissociées en des suspensions de cellules uniques vivantes, que ce soit de manière manuelle ou par digestion enzymatique, ce qui empêchait une analyse par FACS. Une technique basée sur une RT-PCR (Immunoscope®) a été utilisée à la place, pour rechercher les ARNm codant pour des marqueurs spécifiques des cellules NKT. Puisqu'une chaîne TCR Vα14-Jα281 presque invariante, avec un CDR3 de 10 acides aminés de long, est la signature de cellules NKT murines (Lantz et al, 1994), les ADNc codant pour Vα14 préparés à partir d'échantillons contrôles et expérimentaux ont été amplifiés par PCR et analysés pour la diversité de taille de CDR3 par extension d'amorces en utilisant soit des amorces spécifiques de Cα soit des amorces spécifiques de Jα281. Les amorces Cα spécifiques ont produit des signaux représentatifs de la diversité des chaînes α Vα14⁺ des échantillons. Plusieurs pics ont été détectés dans les noyaux lymphatiques drainant le site d'injection chez les souris contrôles et immunisées (figure 1B, **a** et **c**). La distribution gaussienne globale de la taille de CDR3 était altérée par un pic prédominant correspondant à une longueur de CDR3 de 10 acides aminés et contenant des chaînes TCR α réarrangées Vα14-Jα281 (figure 1B, **b** et **d**), incluant ainsi les cellules NKT. Aucun signal n'a été détecté dans la peau et les muscles disséqués au site d'injection du PBS (figure 1B, **e** et **f**). En revanche, les ADNc codant pour Vα14 préparés à partir des structures de type granulomateuse au jour 7 ont conduit à un pic unique avec une taille de CDR3 de 10 acides aminés en utilisant soit les amorces Jα spécifiques soit les amorces Cα spécifiques (figure 1B, **g** et **h**). Le séquençage direct des produits PCR Vα14-Cα, réalisé à partir de ces lésions, a permis d'identifier les chaînes TCR α spécifiques des cellules NKT. Les quatre substitutions nucléotidiques affectant le codon 31 rendent compte de la diversité des chaînes Vα14-Jα281 présentes dans les cellules T infiltrantes. Ainsi, contrairement aux cellules des noyaux lymphatiques qui contiennent beaucoup de cellules T Vα14+ conventionnelles, toutes les cellules Vα14⁺ recrutées dans les structures de type granulomateuse au jour 7 sont des cellules NKT. Un pic unique Vα14-Cα avec un CDR3 de 10 acides aminés de long et contenant le segment Jα281 a été détecté au jour 3 suivant l'injection (figure 1B, **i** et **j**) et a ensuite été identifié comme spécifique des cellules NKT, en utilisant une sonde clonotypique.

Par conséquent, les cellules NKT sont recrutées aux stades les plus précoces de l'infiltration cellulaire dues à l'injection de parois cellulaires déprotéinisées mycobactériennes, et représentent les seules cellules Vα14 T présentes dans les lésions au jour 7.

L'utilisation de la même technique RT-PCR a permis aux auteurs de la présente invention de détecter les chaînes β réarrangées utilisant les segments Vβ8.1, 7, 3, 5,2, 10. Seules de rares cellules T CD4⁺ α/β conventionnelles peuvent infiltrer les lésions de type granulomateuse, puisqu'aucun ARNm codant pour CD8 et peu d'ARNm codant pour CD4 ont pu être détectés en utilisant les amorces appropriées. Finalement, les résultats de l'analyse PCR utilisant des amorces spécifiques des Igµ et de TCR γ/δ indiquent la présence de nombres significatifs de cellules B et de cellules T *γ*/*δ* utilisant en particulier les segments Vδ5 et Vδ6.

### 3. Implication des cellules T α/β dans la réponse granulomateuse à des parois cellulaires déprotéinisées de mycobactéries.

Les réponses granulomateuses sont le résultat de processus multi-cellulaires à plusieurs étapes. Afin d'évaluer la possible implication des cellules T α/β dans le processus granulomateux, les auteurs de la présente invention ont immunisé des souris génétiquement déficientes en plusieurs sous-groupes de cellules T.

On a injecté à des souris Jα281 -/- (Cui et al, 1997) déficientes en cellules NKT, mais dans lesquelles les cellules T conventionnelles et les cellules NK se développent normalement, des parois cellulaires de mycobactéries déprotéinisées. Dans 5 sur 7 des souris ayant subi l'injection, on a observé au site de l'injection aucune lésion de type granulomateuse mais plutôt un petit infiltrat aplati. Chez les deux autres souris, un infiltrat cellulaire structuré minuscule, presque exclusivement composé de macrophages s'est développé (figure 2A, **a** à **d**). Dans aucun des infiltrats, on ne trouve d'ARNm codant pour Vα14 (figure 2B, **a** et **b**) ni de réarrangement VβCβ. Un profil gaussien Vα14-Cα a été obtenu à partir des noyaux lymphatiques chez les souris Jα281 -/- immunisées, indiquant un usage normal du segment Vα14 par les cellules T conventionnelles en absence de cellules NKT (figure 2B, **c** et **d**).

Selon l'analyse RT-PCR aucune chaîne µ n'a été détectée et des chaînes γ TCR réarrangées ont été trouvées. Comme contrôle, les six souris Jα281 +/-, auxquelles on a injecté des parois cellulaires de mycobactéries déprotéinisées ont donné les mêmes résultats que les souris C57BL/6 +/+ (figure 2A, **e** à **g** ; figure 2B, **e** à **h**). La formation d'une lésion de type granulomateuse assez grosse requiert donc la présence de cellules NKT.

Les auteurs de la présente invention ont ensuite examiné la possible implication des cellules T CD4+ conventionnelles. Chez les souris CMH II -/- qui sont déficientes en cellules T CD4+ conventionnelles, mais dans lesquelles les cellules NKT se différencient normalement, l'injection de parois cellulaires de mycobactéries déprotéinisées a conduit au développement de lésions de type granulomateuse comme chez les animaux C57BL/6 +/+ (figure 2A, **h** à **j**). D'après l'analyse Immunoscope des échantillons, la chaîne TCR α invariante Vα14-Jα281 a été la seule chaîne Vα14 détectée (figure 2B, **i** et **j**). Les profils Vα14-Cα et Vα14-Jα281 dans les noyaux lymphatiques des souris de classe II -/- sont les mêmes que chez les animaux C57BL6 +/+ (figure 2B, **k** et **I**). De plus, le répertoire TCR β des cellules présentes dans les lésions au jour 7 des cellules CMH II -/-, a montré le même biais dans l'usage Vβ que chez les souris C57BL/6 +/+. En outre, les ARNm codant pour la chaîne µ de l'Ig et pour les chaînes TCR γ/δ ont été détectés comme chez les souris C57BL6 +/+.

Ainsi, les divers types cellulaires présents dans les lésions de type granulomateuse induites par de parois cellulaires mycobactériennes s'assemblent en une structure hautement organisée en l'absence de cellules T CD4⁺ mais ne s'organisent pas en l'absence de cellules NKT.

L'absence d'ARNm codant pour CD8 exclut un rôle prépondérant pour les cellules T CD8⁺ conventionnelles. Le rôle des molécules CD1 dans le processus granulomateux a été évalué en utilisant des souris β2m -/-, qui sont dépourvues de cellules NKT dépendantes de CD1. De larges lésions de type granulomateuse se sont développées. Cependant, leur contenu en lymphocytes différait de manière marquée de celui observé dans les structures de type granulomateuse induites chez les souris C57BL/6 +/+ : d'après les RT-PCR et l'analyse Immunoscope, les ARNm codant pour les chaînes TCT-β et TCR Vα14⁺, le CD4, le CD8, et les chaînes µ des IgM n'étaient pas détectables. En revanche, les ARNm codant pour les chaînes γ/δ du TCR étaient présents comme chez les souris C57BL6 +/+.

Les changements phénotypiques dus à l'altération du gène β2m préviennent donc l'accumulation des lymphocytes B et des cellules T α/β dans les lésions causées par des parois cellulaires mycobactériennes déprotéinisées, et préviennent également l'accumulation dans la lésion de cellules NKT rares, détectées dans les noyaux lymphatiques des mêmes animaux en utilisant une sonde clonotypique.

### 4. L'activité de recrutement des cellules NKT est associée à des glycolipides glycobactériens, plus particulièrement aux PIM.

Afin d'identifier les composants non peptidiques mycobactériens responsables de l'accumulation des cellules NKT, le matériau a été fractionné et l'activité de recrutement des cellules NKT des différentes fractions a été évaluée *in vivo.* Les quantités relatives de cellules NKT présentes au jour 7 au site d'injection ont été déterminées par RT-PCR et analyse Immunoscope. Pour éviter de possibles différences dans le comportement *in vivo* des molécules injectées (par exemple, des différences dans le taux de diffusion ou dans le processus d'intemalisation spécifique) le matériau injecté a été conjugué à un véhicule neutre l'hydroxyde d'aluminium (Alu-Gel-S, Serva). Les souris C57BL6 +/+ à qui on a injecté par voie sous-cutanée, de l'Alu-Gel-S sous forme de particules de 0,25 mg, ont développé un infiltrat faible avec un ARNm Vα14-Jα281 à peine détectable (figure 3).

Les lipides bruts des fractions cellulaires H37Rv (fraction I) adsorbés sur l'Alu-Gel-S, ont provoqué de larges infiltrats cellulaires organisés avec un noyau formé par le véhicule et des neutrophiles, entouré d'une bordure épaisse de macrophages et de lymphocytes. La formation de ces structures s'est également avérée dépendre de la présence de cellules NKT, puisqu'elles ne se formaient pas chez les souris Jα281 -/-. Cependant, et contrairement aux structures induites par les parois cellulaires déprotéinisées, les infiltrats cellulaires organisés induits par les glycolipides bruts pouvaient être dissociés en des cellules uniques vivantes, permettant ainsi l'identification directe par analyse FACS des cellules NKT dans les infiltrats. Les cellules mortes et les macrophages ont été éliminés par fractionnement Ficoll. La plupart des 2.10⁶ cellules vivantes qui ont été recueillies à partir de chaque granulome ont été identifiées comme étant des neutrophiles. La figure 4 montre que toutes les autres cellules étaient des leucocytes CD45⁺. Aucune cellule B CD19⁺B220⁺ n'a été trouvée. Toutes les lymphocytes T α/β étaient Vα14⁺, TCR β^{int} (expression intermédiaire) et NK1.1.⁺, c'est à dire des cellules T NK1.1⁺. Aucune cellule CD8⁺ et de rares cellules CD4⁺ ont été détectées. Environ 0,3 % étaient des cellules NK non-T et environ 0,1 % étaient des cellules T γ/δ. Les analyses par RT-PCR et Immunoscope ont confirmé l'absence de cellules B et la présence de chaînes TCR γ/δ réarrangées, dans les infiltrats causés par l'injection de fraction I. Presque toutes les cellules T infiltrant les lésions causées par les lipides bruts étaient donc des cellules NKT CD4⁻ CD8⁻. Par analyse RT-PCR, l'infiltrat induit par la fraction I s'est avéré contenir jusqu'à 200 fois plus d'ARN Vα14-Jα281 que les cellules recrutées au site d'injection d'Alu-Gel-S (figure 3). Alors qu'elle est capable d'activer les cellules T γ/δ chez l'homme (Constant et al, 1994), la fraction non lipidique (fraction II) était inefficace pour recruter les cellules NKT (figure 3). L'immuno-adjuvant Muramyl-di-peptide (MDP, Sigma), un composant des parois cellulaires mycobactériennes, a conduit à un infiltrat cellulaire sans augmentation de l'ARNm de Vα14-Jα281 en comparaison avec l'Alu-Gel-S seul (figure 3). La fraction I a été fractionnée et l'activité de recrutement de cellules NKT des fractions a été suivie par RT-PCR. Presque toute l'activité a été détectée dans les fractions contenant des PIM (fractions III, IV, V et VII) (figure 3), et l'aire du pic Vα14-Jα281 était proportionnel à la quantité de matériel injecté. D'après l'analyse des fractions réactives par TLC par "Flash LC®" (Merck) avec un gel "Kieselgel G60 silicagel", les fractions les plus actives dans le recrutement des cellules NKT étaient celles contenant des PIM hautement polaires dont la structure, par analyse de spectrométrie de masse, est compatible avec des PIM comprenant 4 à 6 motifs mannose.

Afin de déterminer si les cellules NKT s'accumulent de manière aléatoire dans les infiltrats induits par les PIM 4-6, une technique de PCR semi-quantitative dérivée de l'Immunoscope a été utilisée pour définir leur répertoire TCRβ et le comparer au répertoire des cellules NKT de foie choisi de manière arbitraire comme représentatif des cellules NKT périphériques (Watanabe et al, 1995) (figure 5). On a confirmé que les cellules NKT de foie avaient un usage Vβ restreint vers Vβ8 et Vβ7 en comparaison des cellules T conventionnelles. Chaque famille de réarrangement Vβ-Cβ montrait une distribution gaussienne de longueur de CDR3, indicatrice d'un répertoire TCR β polyclonal et diversifié.

En revanche, les cellules NKT présentes dans l'infiltrat causé par l'injection de PIM 4-6 montraient un usage Vβ limité de manière différente (figure 5). En outre, les réarrangements associés à une longueur CDR3 donnée (tels que des réarrangements Vβ8.1-Cβ avec une longueur de CDR3 de 9 acides aminés comme dans la figure 5) et trouvés en large excès en comparaison avec la distribution gaussienne observée dans les contrôles, ont révélé une distribution oligoclonale des cellules NKT dans les infiltrats. La même limitation dans l'usage Vβ, associée à des distributions oligoclonales a été observée chez toutes les souris étudiées, mais la longueur des régions CDR3 variait selon les animaux.

### BIBLIOGRAPHIE

- Bendelac, A., Lantz, O., Quimby, M. E., Yewdell, J. W., Bennink, J. R. & Brutkiewicz, R. R. (1995) *Science* **268**, 863-865.
- Bendelac, A., Rivera, M. N., Park, S. H. & Roark, J. H. (1997) *Ann.Rev.Immunol*. **15,** 535-562.
- Brossay, L., Chioda, M., Burdin, N., Koezuka, Y., Casorati, G., Dellabona, P. & Kronenberg, M. (1998) *J. Exp.Med.* **188,** 1521-1528.
- Brossay, L., Naidenko, O., Burdin, N., Matsuda, J., Sakai, T. & Kronenberg, M. (1998) *J. Immunol*. **161,** 5124-5128.
- Burdin, N., Brossay, L., Koezuka, Y., Smiley, S. T., Grusby, M. J., Gui, M., Taniguchi, M., Hayakawa, K. & Kronenberg, M. (1998) *J. Immunol.* **161,** 3271-3281.
- Cibotti, R., Cabaniols, J. P., Pannetier, C., Delarbre, C., Vergnon, I., Kaneliopoulos, J. M. & Kourilsky, P. (1994) J. *Exp.Med.* **180**, 861-872.
- Constant, P., Davodeau, F., Peyrat, M.-A., Poquet, Y., Puzo, G., Bonneville, M. & Fournier, a. J.-J. (1994) *Science* **264,** 267-270.
- Cosgrove, D., Gray, D., Dierich, A., Kaufman, J., Lemeur, M., Benoist, C. & Mathis, D. (1991) *Cell* **66,** 1051-1066.
- Cui, J., Shin, T., Kawano, T., Sato, H., Kondo, E., Toura, I., Kaneko, Y., Koseki, H., Kanno, M. & Taniguchi, M. (1997) *Science* **278,** 1623-6.
- Emoto, M., Emoto, Y. & Kaufmann, S. H. (1997) *Eur. J. Immunol.* **27,** 183-188.
- Emoto, Y., Emoto, M. & Kaufmann, S. H. (1997) *Inf. & Imm*. **65,** 5003-5009.
- Enomoto, A., Nishimura, H. & Yoshikai, Y. (1997) *J. Immunol*. **158,** 2268-2277.
- Flesch, I. E. A., Wandersee, A. & Kaufmann, S. H. E. (1997) *J. Immunol*. **159,** 7-10.
- Hammond, K. J. L., Poulton, L. D., Palmisano, L. J., Silveira, P. A., Godfrey, D. I. & Baxter, A. G. (1998) *J. Exp.Med*. **187,** 1047-1056.
- Kawano, T., Cui, J., Koezuka, Y., Toura, I., Kaneko, Y., Motoki, K., Ueno, H., Nakagawa, R., Sato, H., Kondo, E., Koseki, H. & Taniguchi, M. (1997) *Science* **278,** 1626-1629.
- Kawano, T., Cui, J., Koezuka, Y., Toura, I., Kaneko, Y., Sato, H., Kondo, E., Harada, M., Koseki, H., Nakayama, T., Tanaka, Y. & Taniguchi, M. (1998) *Proc. Natl. Acad. Sci. USA* **95,** 5690-5693.
- Koller, B. H., Marrack, P., Kappler, J. W. & Smithies, O. (1990) *Science* **248,** 1227-1230.
- Koseki, H., Asano, H., Inaba, T., Miyashita, N., Moriwaki, K., Lindahl, K. F., Mizutani, Y., Imai, K. & Taniguchi, M. (1991) *Proc. Natl* Acad. Sci. U.S.A. **88,** 7518-7522.
- Lantz, O. & Bendelac, A. (1994) *J. Exp.Med*. **180,** 1097-1106.
- Levraud, J. P., Pannetier, C., Langlade-Demoyen, P., Brichard, V. & Kourilsky, P. (1996) *J. Exp.Med.* **183,** 439-449.
- Mieza, M. A., Itoh, T., Cui, J. Q., Makino, Y., Kawano, T., Tsuchida, K., Koike, T., Shirai, T., Yagita, H., Matsuzawa, A., Koseki, H. & Taniguchi, M. (1996) *J. Immunol*. **156,** 4035-4040.
- Ortaldo, J. R., Winckler-Pickett, R., Mason, A. T. & Mason, L. H. (1998) *J.Immunol*. **160,** 1158-1165.
- Pannetier, C., Levraud, J.-P., Lim, A., Even, J. & Kourilsky, P. (1997) in : *The T-cell receptor: selected protocols and applications.* Okseznberg, J.R., Wang, L. and Jeffery, J.-Y. Y., Eds. (Chapman and Hall, New York), pp. 287-324.
- Regnault, A., Levraud, J. P., Lim, A., Six, A., Moreau, C., Cumano, A. & Kourilsky, P. (1996) *Eur. J. Immunol.* **26,** 914-921.
- Schofield, L., McConville, M. J., Hansen, D., Campbell, A. S., Fraser-Reid, B., Grusby, M. J. & Tachado, S. D. (1999) *Science* **283,** 225-229.
- Takeda, K., Seki, S., Ogasawara, K., Anzai, R., Hashimoto, W., Sugiura, K., Takahashi, M., Satoh, M. & Kumagai, K. (1996) *J. Immunol*. **156,** 3366-3373.
- Watanabe, H., Miyaji, C., Kawachi, Y., liai, T., Ohtsuka, K., Iwanage, T., Takahashi-Iwanaga, H. & Abo, T. (1995) *J. Immunol.* **155,** 2972-2983.
- Wilson, S. B., Kent, S. C., Patton, K. T., Orban, T., Jackson, R. A., Exley, M., Porcelli, S., Schatz, D. A., Atkinson, M. A., Balk, S. P., Strominger, J. L. & Hafler, D. A. (1998) *Nature* **391,** 177-181.

## Revendications

1. Utilisation d'au moins un phosphatidylinositolmannoside (PIM) et/ou au moins une cellule NKT (lymphocyte T « Natural Killer ») activée par un PIM pour la fabrication d'une composition pharmaceutique destinée au traitement d'une maladie pouvant être améliorée ou évitée par l'induction d'une réponse NKT *in vivo*.

2. Utilisation selon la revendication 1, dans laquelle ledit PIM est d'origine mycobactérienne.

3. Utilisation selon la revendications 1 ou 2, dans laquelle ledit PIM comporte 4, 5 ou 6 motifs mannose.

4. Utilisation selon l'une quelconque des revendications 1 à 3, dans laquelle la réponse NKT est une réaction immunitaire du type granulomateuse.

5. Utilisation selon l'une quelconque des revendications 1 à 4, dans laquelle la maladie à traiter est un cancer.

6. Utilisation selon la revendication 5, dans laquelle le cancer est un mélanome.
